(19) Europäisches Patentamt
European Patent Office
Office européen des brevets

(11) Publication number: **0 450 318 A1**

# (12) EUROPEAN PATENT APPLICATION

(21) Application number: **91103225.8**

(51) Int. Cl.5: **C07C 15/48**, C07C 5/00

(22) Date of filing: **04.03.91**

(30) Priority: **26.03.90 US 498679**

(43) Date of publication of application:
**09.10.91 Bulletin 91/41**

(84) Designated Contracting States:
**CH DE FR GB IT LI**

(71) Applicant: **HERCULES INCORPORATED**
**Hercules Plaza**
**Wilmington Delaware 19894-0001(US)**

(72) Inventor: **Carosino, lawrence Enrico**
**512 Woodside Avenue/Woodside Hills**
**Wilmington, Delaware 19809(US)**
Inventor: **Herak, David Charles**
**3236 Brookline Drive**
**Wilmington, Delaware 19808(US)**

(74) Representative: **Lederer, Franz, Dr. et al**
**Lederer, Keller & Riederer, Patentanwälte,**
**Lucile-Grahn-Strasse 22**
**W-8000 München 80(DE)**

## (54) An improved synthesis of diethynylbenzene.

(57) A process for making diethynylbenzene by a "one-pot" reaction in which mixed isomers of divinylbenzene (DVB) and bromine are combined in a solvent consisting of sulfolane, at a rate that does not cause the temperature of the reaction to rise above 50° C., the intermediate brominated product is dehydrobrominated, and diethynylbenzene is separated from the dehydrobromination reaction mixture, is disclosed.

EP 0 450 318 A1

This invention relates to a process for preparing diethynylbenzenes by brominating divinylbenzene, dehydrobrominating an intermediate product, and recovering diethynylbenzene.

It is known that ethynylbenzenes may be produced by means of a sequence of halogenation and dehydrohalogenation reactions, for instance from an article by N.N Lebedev, et. al. in 84 Chem. Abstracts 105109e 1976), who prepared ethynyl derivatives of benzene from the chlorination and dehydrochlorination of technical grade divinylbensene (52% of a mixture of meta and para divinylbenzene (DVB) and 34% of a mixture of meta and para ethylstyrene). The DVB was chlorinated in dimethyl formamide as a solvent, and the intermediate product was dehydrochlorinated to diethynylbenzene in 45% yield with potassium hydroxide, using a different solvent, isopropanol.

It is also known, from an article by A. S. Hay, in the Journal of Organic Chemistry 25 (1960) 637, that meta and para bis (alpha, beta dibromoethynyl) benzenes can be prepared by brominating commercial divinylbenzene (40% DVB isomers) in chloroform, separating the isomers by molecular distillation, and dehydrobrominating them with potassium (sic) in a different solvent, tertiary butanol. (Obviously, potassium hydroxide as the alkali was intended). The isomers were respectively isolated by precipitation with water, or ether extraction and distillation.

Since the use of chloroform, which is believed to be a carcinogen, presents unacceptable environmental problems in the workplace, the bromination/dehydrobromination process of Hay is not available for industrial use, for instance in the manufacture of polyarylacetylene resins. Those resins are in demand because of their superior properties, but their industrial development has been hampered by the absence of an economically practical process for preparing the diethynylbenzene monomers. It is noted that both of the known processes mentioned are not what can be referred to as "one pot" processes, since an intermediate product separation and a change of solvent are required.

There is therefore a clear need for an inexpensive one-pot process for synthesizing diethynylbenzene monomers, that produces a high yield of product with a relatively low input of bromine.

According to the invention, a process for making diethynylbenzene in which mixed isomers of divinylbenzene (DVB) and bromine are combined in a solvent, the intermediate brominated product is dehydrobrominated, and diethynylbenzene is separated from the dehydrobromination reaction mixture, is characterized in that the solvent is sulfolane, the concentration of the divinylbenzene in the solvent is 20 to 50%, sufficient bromine is added to the reaction mixture to react with the olefinic unsaturation in the divinylbenzene, and the bromine and the divinylbenzene are combined at a rate that does not cause the temperature of the reaction to rise above 50 °C.

The present invention provides an inexpensive "one-pot" procedure for the synthesis of diethynylbenzene at high yields. The following equations summarize the chemistry of the invention.

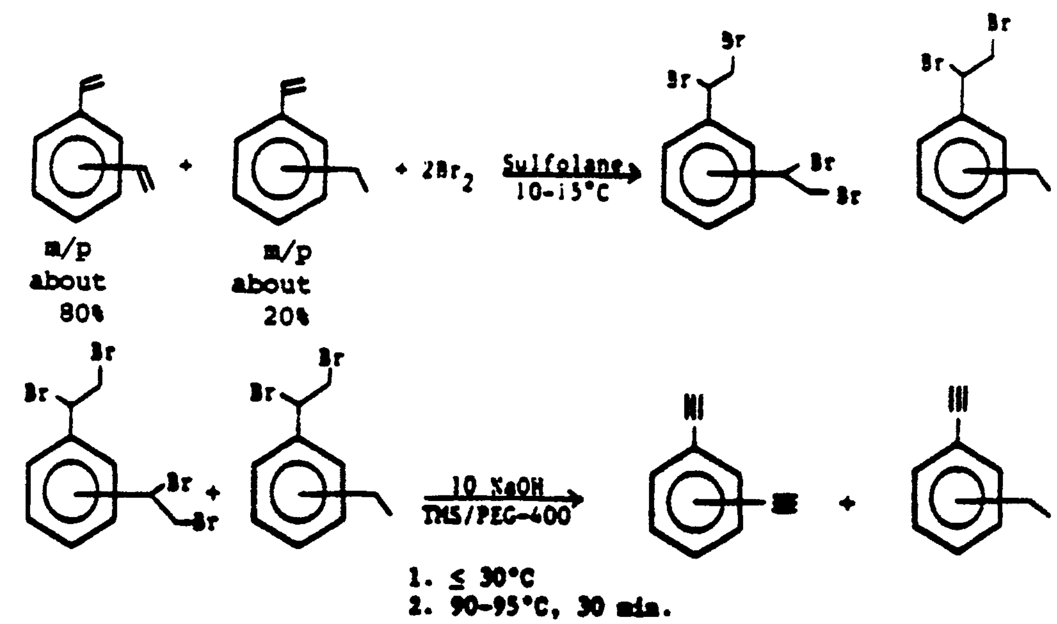


One commercially available form of sulfolane (tetrahydrothiophene 1,1-dioxide) is produced by Phillips Petroleum Company and distributed by Aldrich Chemical Company, Inc., as "tetramethylene sulfone", with

a purity level of 99%. Its specific gravity is 1.261.

The reaction of bromine with divinylbenzene in sulfolane is instantaneous and exothermic, and may be carried out at from 0° C to 50° C. It is accompanied by a steady low level elimination of HBr. Preferably, the rate of combination is controlled so that the temperature is maintained in the range from 10-15° C, to minimize any tendency to over-brominate the DVB. With efficient cooling, and depending on the size of the vessel, three to four hours or more are required to carry out the bromination reaction and to keep the polymerization side reaction to a minimum. When carried out in the laboratory on a small scale, 5 liters or less, yields of diethynylbenzene in the range of 70-75% are obtained.

The heat of reaction of the bromination step in sulfolane (20% concentration of DVB by weight at 20° C) is exothermic by 51-52 KCal./mole. The estimated adiabatic temperature rise for the entire amount of bromine is about 190° C. In large scale laboratory brominations, no more than a quarter of the total charge of bromine should be placed in an addition funnel at one time as a safety precaution, thus reducing the potential adiabatic temperature rise caused by an excessive addition rate to the range of about 45 to 50° C.

Preferably, the total amount of divinylbenzene added to the sulfolane is between 20-50 percent by weight of the solvent, more preferably 25% to 30% by weight. A higher concentration, up to 40 or 50%, can be used, but it requires more control of agitation and temperature and extends the bromination period, and may actually decrease the yield of diethynylbenzene. A small excess of bromine should be present throughout the course of the reaction.

Preferably also, the total amount of bromine added is not more than 10 percent over the stoichiometric amount necessary to react with all of olefinic unsaturation of the divinylbenzene. The preferred concentration of bromine can be calculated from the concentration of olefinic unsaturation of the DVB monomer (0.65 ml Br per gram of DVB; the density of bromine at normal room temperature is 3.06 g/ml). Preferably, about 2% excess bromine is maintained in the reaction mixture. An excess of bromine over 10% is not desirable, since in the subsequent dehydrobromination step, excess bromine would form hypobromite, an undesirable oxidizing agent, after reaction with alkali.

Although all of the divinylbenzene may be placed in the reaction vessel with the sulfolane solvent, followed by gradual addition of bromine, regulated as indicated above to maintain a desirable reaction temperature, step-by-step addition of the DVB in increments not exceeding 5 percent by weight of the total addition, accompanied by corresponding incremental additions of bromine, regulated to maintain the reaction temperature in the preferable range of 15 to 30° C, is preferred.

Preferably, commercial divinylbenzene such as that available from the Dow Chemical Company, that can be between 50% to 80% of a mixture of meta and para isomers of divinylbenzene, more preferably about 75-80% and most preferably 78 to 80% divinylbenzene isomers, is used, to maximize the yield of diethynylbenzene. A typical sample of DVB suitable for the process of the invention has the following composition:

| | | | |
|---|---|---|---|
| Divinylbenzene | 79% | meta | 54.7% |
| | | para | 25.2% |
| Ethylstyrene | 18.0% | meta | 10.5% |
| | | para | 7.5% |
| Other | 2.1% | | |

The next step is to react the brominated divinylbenzene product with a dehydrobromination caustic agent such as sodium hydroxide or potassium hydroxide. Preferably, an excess of the agent is added to total about 50-250 mole% more than the amount required to remove all organically bound bromine from the brominated divinylbenzene product, as the corresponding halide salt. Addition of the caustic agent is continued until the excess of the agent is present and then the reaction with the caustic agent is completed by heating the dehydrobrominated product to a temperature between about 90 to about 100° C for a period of about 1/2 to 4 hours. The excess dehydrobromination caustic agent and the halide salts are then removed and a layer is isolated comprising the sulfolane and diethynylbenzene. The final step in the process is to recover the diethynylbenzene from the isolated layer.

Preferably, the dehydrobromination caustic agent is either sodium hydroxide or potassium hydroxide,

but other conventional dehydrobromination caustic agents such as tetramethyl ammonium hydroxide or trimethylethanolammonium hydroxide (choline hydroxide) may be used. When sodium hydroxide is used, it is preferably about 50% aqueous (about 45-60% aqueous when potassium hydroxide is used).

Before dehydrobromination, the reaction mixture from the bromination step is preferably treated with a phase transfer agent at a concentration level of about 2% of the total weight of the brominated DVB plus sulfolane. Failure to add a phase transfer agent will result in a considerable reduction in the rate of dehydrobromination and a buildup in the amount of excess caustic agent in the mixture, with the danger of an exothermic reaction. However, since the dehydrobromination reaction proceeds without the phase transfer agent, one can omit the phase transfer agent and either run the reaction at a higher temperature or for a longer period of time, or both.

The phase transfer agent, preferably a polyethylene glycol with $HO-(CH_2CH_2O)_n-CH_2CH_2OH$ as the general formula, in which n is an integer from 1-20, may be added in an amount that totals about 0.5 to 3.0% by weight of the reaction mixture, including the weight of the caustic reagent added in the next step.

Other conventional phase transfer agents, which are discussed, for instance, by Kimura et al, J. Organic Chemistry 47, 2493 (1982) and J. Organic Chemistry 48, 195 (1983) can be used in the dehydrobromination reaction. Examples of such agents are the quaternary ammonium compounds tricaprylmethylammonium chloride, tetrabutylammonium hydrogen sulfate, and benzyltri-n-butylammonium bromide. The most preferred phase transfer agent is polyethylene glycol 400 (PEG-400; Dow Chemical Co.) having an average molecular weight of 400.

Dehydrobromination through addition of a caustic agent, for example, NaOH or KOH, is carried out in two steps. The first step is exothermic and is carried out at 15 to 50° C, (preferably for the best yield at 15 to 30° C) according to the following equation.

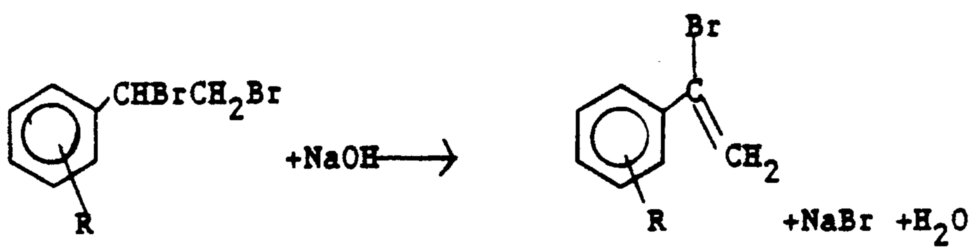


The removal of the first equivalent of hydrogen bromide from all of the brominated species in the mixture was found to be exothermic by 57.1 KCal./mole at 25° C when KOH was the base. (The heat of reaction with NaOH as the base would be expected to be less than when measured for KOH). If the temperature is allowed to climb above about 30° C, a progressive decline in yield of diethynylbenzene can be caused by a side reaction involving hydrolysis of the vinylic bromide to a carbonyl function (which would undergo further complex condensation reactions under strongly alkaline conditions).

After the first stoichiometric equivalent of NaOH has been added to effect the removal of one mole of HBr, the removal of the second mole of HBr becomes endothermic, and the system is heated to 90-100° C for thirty minutes to effect the following reaction.

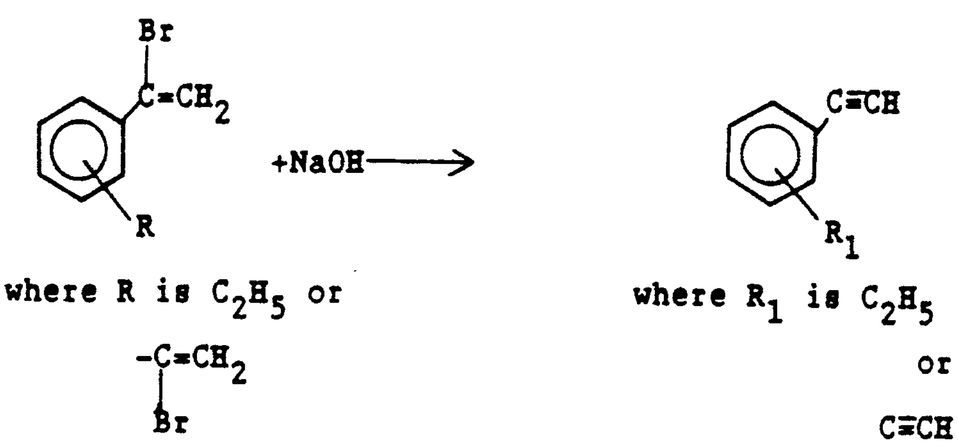

The reaction mixture undergoes profound color changes; from amber to magenta, to purple and eventually to dark brown. After the addition of about one-half of the necessary base to achieve dehydrobromination, the mixture becomes very viscous and has the appearance of an invert emulsion.

The reaction is complete after heating for about thirty minutes at about 95° C, which can be confirmed by following the reaction with high performance liquid chromatography (HPLC), which shows a reaction profile that is virtually unchanged after three hours at that temperature. The mixture is allowed to cool and when agitation is halted at about 80° C , the mixture undergoes phase separation and crystalline sodium bromide settles out of the aqueous layer. The very dark upper layer consists of sulfolane with the diethynylbenzene and residual compounds.

The total bromine analysis of the sulfolane layer typically amounts to only about 3% of the bromine used in the synthesis. More than 90% of the bromine used is converted to sodium bromide. Conversion of a large proportion of organic bromide to sodium bromide is desirable to avoid separation problems with the sulfolane layer caused by emulsion formation.

After the layers separate and while the mixture is at about 80° C, the bottom layer, including most of the precipitated NaBr, is removed, for instance, by means of a vacuum-assisted dip tube. The lower layer and insoluble salts can be drawn off into a filter flask by means of aspirator vacuum.

The final stage of the process is to recover the diethynylbenzene product. In the laboratory the product can be recovered by steam distillation. The product from steam distillation is sufficiently pure for use in catalyzed polymerizations of the type described in U.S. Patents 4,070,333 and 4,097,460. The diethynylbenzene recovery can also be accomplished by vacuum column distillation, which affords a purer product.

Furthermore, diethynylbenzene undergoes highly exothermic polymerization above 100° C. Conventional batch laboratory vacuum distillation of this product should be limited to amounts of 25 g. or less, with adequate safety precautions. An attempt to distill diethynylbenzene, produced by the reaction of 1,3-dibromobenzene with trimethylsilylacetylene (TMSA), disclosed by Neenan et al, J. Organic Chemistry 53, 2489 (1988), resulted in an exothermic reaction and an explosion, and it was stated that the product should be distilled at high vacuum and at temperatures of less than 60° C in well-shielded equipment. (The article confirms that the absence of an inexpensive route to produce the diethynylbenzene monomers has discouraged the full development of polyacetylenic aromatic compounds).

Unless the salt-caustic layer is removed, the temperature of the subsequent steam distillation rises to as high as 120-125° C and slowly drops to about 108-110° C over the duration of the steam distillation. Part of this exotherm is attributable to the heat of dilution of the residual concentrated caustic-NaBr layer. Also, if column distillation is used, the soluble sodium bromide may plug the column if it is not removed.

Part of the reason for the prolonged character of the exotherm is that some of the diethynylbenzene monomer may be lost by polymerization. The exotherm can be reduced to a high of about 108° C, dropping to about 101-103° C at the end of the steam distillation by replacing the NaBr-caustic layer with water. To maintain the highest possible yield of product, it is necessary to avoid metal ion contamination by iron, nickel and other transition metals, which may catalyze the unwanted polymerization of the acetylenic monomer during the steam distillation.

The steam distillate is condensed with ice-bath cooling and toward the end of this step solid para-diethynylbenzene tends to form on the walls. It is convenient for the purposes of separating the product layer to transfer the suspension to a jacketed vessel with a bottom drain. The slurry can be warmed to 40-50° C, and the diethynylbenzene oil is then easily separated. The aqueous layer may be extracted with ethyl acetate or ethyl ether to recover a small additional amount of diethynylbenzene. Yields of product are in the 70 to 75% range.

## EXAMPLE 1

This Example describes a laboratory procedure for producing diethynylbenzene using sodium hydroxide as the caustic agent for the dehydrobromination. A three-necked, five-liter, round-bottom flask was equipped with an air-driven Teflon paddle stirrer, a 250 ml vapor-bypass dropping funnel and a Y-shaped adapter provided with a pot thermometer, a gas inlet side arm and a condenser. The flask was cooled with an ice water bath.

The flask was charged with 1144 ml of anhydrous sulfolane (sold by Aldrich as tetramethylene sulfone and 99% pure). Then 470 grams (3.6 moles) of high purity, divinylbenzene (Dow Chemical Co. molecular weight of about 130 by bromine titration and gas chromatography analysis) was charged to the flask. The mixture was stirred and the flask was cooled to bring the temperature to about 10° C. An initial portion of 150 ml of liquid bromine was charged to the addition funnel and then added to the reaction flask dropwise at a rate to maintain the reaction temperature at a maximum of 15° C. A second portion of liquid bromine in

the amount of 166 ml was charged to the addition funnel and added under the same conditions. A total of 316 ml of bromine was added (967 grams, 6.05 moles, based upon a density of 3.06 ms/ml. for bromine). The reaction of bromine with divinylbenzene was instantaneous and at the completion of the addition the reaction mixture was a light red-amber color.

Then 62.1 grams of Polyethylene Glycol 400 (Dow Chemical Co.) were added to the reaction mixture. The vapor-bypass funnel was rinsed out with 20% aqueous $NaHSO_3$ solution and water and was then charged with 250 ml of 50% aqueous NaOH solution. A total of 2884 grams (about 1900 ml) of 50% NaOH was used for the dehydrobromination, which constitutes about a 200 mole % excess of NaOH. When sufficient NaOH had been added to remove the first equivalent of HBr from the mixture of bromo compounds, the reaction temperature ceased to climb. The remainder of the NaOH was then added rapidly, and heat was applied to the reaction mixture to raise the temperature to about 95° C for thirty minutes.

At this point the reaction was interrupted, the reactor was arranged for steam distillation, and the mixture allowed to cool to about 80° C. The mixture separated into a black organic upper layer and a colorless lower layer containing the excess NaOH and dissolved NaBr. Solid NaBr separated from solution during the cooling. The lower inorganic layer, including most of the insoluble salts, was drawn off with a vacuum siphon dip tube.

The bottom layer was then replaced with 1200-1300 ml of distilled water. The mixture was stirred and heated to over 90° C and steam was passed into the mixture until about four liters of steam distillate had been obtained. The reactor was allowed to cool to room temperature. The dark upper layer of organic material was separated. The lower layer contained some dissolved salts and sulfolane. The steam distillate was composed of a yellow, oily, upper layer of diethynylbenzene and a hazy, lower, aqueous layer. This was separated in a jacketed resin kettle with a bottom drain. In this way the mixture could be heated with warm water to prevent para-diethynylbenzene from crystallizing. The recovered diethynylbenzene oil was dried over anhydrous sodium sulfate. On a five-liter scale, the reaction described in Example 1 affords about 300-350 grams of product or about a 70-75% yield.

The above procedure was repeated, but with a more rapid addition to allow the adiabatic temperature rise in the bromination step to reach the range of about 45 to 50° C, and with a reduction in yield of about 10%, and producing a satisfactory product.

EXAMPLE 2

In Example 2, diethynylbenzene was prepared from divinylbenzene using potassium hydroxide in place of sodium hydroxide.

A three-necked, five-liter, round-bottom flask, equipped with an air-driven teflon paddle stirrer, a 250 ml vapor-bypass dropping funnel and a Y adapter provided with a pot thermometer, a gas inlet side arm and a condenser was charged with 700 ml of tetramethylene sulfone (about 880 grams) (Aldrich, 99%) and 274 grams (2.11 mole) of divinylbenzene (Dow Chemical Co.). The mixture was stirred and cooled to 20° C with an ice water bath. Liquid bromine was added dropwise over 1 1/2 hours so as to maintain the temperature at 20-28° C with no loss of $Br_2$ vapor from the system. A total of 634 gms. (3.96 moles) of bromine was added, and the dark reaction mixture was allowed to stand overnight.

The mixture was stirred an additional two hours at room temperature, then 41.6 grams of polyethylene glycol (PEG-400; Aldrich) was added, (about 2% of the mixture by weight) and the stirred mixture was cooled to 17-18° C. To this was then added dropwise a 60% KOH solution (1375 grams of KOH pellets (85% KOH) in 916 grams distilled water, 20.9 moles KOH, 2290 grams of solution amounting to a 160 mole % excess) over about one hour while maintaining the temperature in the 18-27° C range with ice-bath cooling. The reaction exotherm is confined to the reaction of the first half of the KOH addition, which coincides with the removal of the first two moles of HBr. The removal of the second two moles of HBr leading to the acetylenic formation is endothermic.

The reaction mixture was then heated to 80° C for two hours. The system was rearranged for steam distillation, and the reaction mixture was then steam distilled at about 110° C for about 1 1/2 hours to yield about two liters of distillate. The top layer of diethynylbenzene was separated (146.7 grams) and was dried over $Na_2SO_4$ in a dark bottle. The remainder of the steam distillate (aqueous layer) was extracted with 250 ml of ether and dried over $Na_2SO_4$.

When evaporated, 2.1 grams of product was yielded. Steam distillation was continued to yield another liter of steam distillate which was extracted with 250-350 ml of ether. The dried ether extract ($Na_2SO_4$) was evaporated to yield 11 grams of product.

The solution in the flask, upon standing overnight at ambient temperature, separated into two layers. The bottom aqueous layer was separated and discarded; the remainder was steam distilled to afford two

more distillates of about one liter each. The first of these was extracted with 300 ml of ether and after drying over $Na_2SO_4$, yielded 18.93 grams of oily product. The pot residue was discarded. The total of product fractions totalled 182.6 grams, corresponding to a yield of 68.7%. An analysis of the principal fraction by gas liquid chromatography produced the following results:

| Constituent | Aera Percent |
|---|---|
| 3-ethylphenylacetylene | 11.5 |
| 4-ethylphenylacetylene | 12.0 |
| m-diethynylbenzene | 48.5 |
| p-diethynylbenzene | 22.8 |
| Other | 5.3 |

The above procedure was repeated, but with the reaction temperature of the exothermic phase of the dehydrobromination step being allowed to rise to about 50 °C, and with a reduction in yield of about 10%, and producing a satisfactory product.

EXAMPLE 3

In Example 3, diethynylbenzene was prepared from divinylbenzene using the same chemistry as in Example 1, but the addition method of bromine and divinylbenzene was altered. Instead of adding all the divinylbenzene to the pot initially, both the divinylbenzene and bromine were added in increments using dropping funnels. A jacketed, five-necked, 12-liter, round-bottom flask was equipped with a Teflon paddle stirrer, two 250 ml vapor-bypass dropping funnels, a pot thermometer, and a gas inlet tube. The flask was cooled using a refrigeration unit that pumped an ethylene glycol/water mixture through the jacket at about 5 °C.

The 12-liter flask was charged with 1500 gm of anhydrous sulfolane (tetramethylene sulfone, Phillips 99.9% pure). Then 39 ml (36 gm) of divinylbenzene (Dow Chemical Co. high purity) was charged to the stirred reactor and the contents were cooled to 10 °C. It was necessary to add some DVB to the sulfolane (M.P. = 27 °C) before cooling to 10 °C to avoid freezing of the sulfolane. The dropping funnels were charged with bromine and divinylbenzene. 25 ml of bromine were added to the flask dropwise at a rate to maintain the reaction temperature at 15 °C. Then a 39 ml addition of divinylbenzene was made from the addition funnel to the reaction flask. Another 25 ml increment of bromine was added to the flask dropwise to maintain the temperature at 15 °C. This procedure was repeated until 14 increments each of divinylbenzene and bromine had been added to the reactor. The total amounts added were 546 ml divinylbenzene (500 gm, 3.8 moles) and 350 ml bromine (1092 gm, 6.8 moles).

The reaction mixture was then dehydrobrominated using the same technique described in Example 1. Polyethylene Glycol 400 (61.4 gm) was added to the mixture and then a 50 wt. % NaOH solution was added. The temperature was maintained at 30 °C during the exothermic phase of the reaction by controlling NaOH addition and then raised to 95 °C for a 30 min. hold. The total mass of 50 wt. % NaOH added was 3200 gm, which represents a 200 mole % excess. The mixture was then cooled to 80 °C, and the black organic upper layer was separated from the caustic layer and precipitated NaBr by decanting and using a separatory funnel.

The organic layer was then mixed with 500 ml of distilled water in a separate 12-liter flask which was equipped for steam distillation. The mixture was stirred and steam was passed into the mixture. The distillate was condensed and collected. The condensed distillate was composed of a yellow, oily, upper layer of diethynylbenzene and a hazy lower aqueous layer. This mixture was separated in a separatory funnel. The product collected weighed 362 gm, which represents a 76% yield. This yield was about 10-15% higher than for similar diethynylbenzene synthesis procedures in the 12-liter equipment where all the divinylbenzene was mixed with the sulfolane before any bromine was added, such as in Example 1.

The above procedure was repeated, but with continuous addition of DVB and bromine via terestalic pumps that individually delivered a stoichiometric ratio of each reactant, providing a good yield that was

less sensitive to fluctuations in temperature.

**Claims**

1. A process for making diethynylbenzene in which mixed isomers of divinylbenzene and bromine are combined in a solvent, the intermediate brominated product is dehydrobrominated, and diethynylbenzene is separated from the dehydrobromination reaction mixture, characterized in that the solvent is sulfolane, the concentration of the divinylbenzene in the solvent is 20 to 50% by weight , sufficient bromine is added to the reaction mixture to react with the olefinic unsaturation in the divinylbenzene, and the bromine and the divinylbenzene are combined at a rate that does not cause the temperature of the reaction to rise above 50 ° C.

2. A process for making diethynylbenzene as claimed in claim 1, further characterized in that the divinylbenzene and bromine are combined at a rate that maintains the temperature of the reaction in the range from 10-15 ° C.

3. A process for making diethynylbenzene as claimed in claim 1 or 2, further characterized in that the total amount of divinylbenzene added to the sulfolane is between 25% to 30% percent by weight of the solvent.

4. A process for making diethynylbenzene as claimed in any one of the preceding claims, further characterized in that an excess of bromine is present throughout the course of the reaction and the total amount of bromine added to the reaction mixture is not more than 10 percent over the stoichiometric amount necessary to react with all of olefinic unsaturation of the divinylbenzene.

5. A process for making diethynylbenzene as claimed in any one of the preceding claims, further characterized in that the divinylbenzene and bromine are combined simultaneously by addition of the DVB and bromine either continuously or in increments of the DVB each followed by a corresponding increment of bromine.

6. A process for making diethynylbenzene as claimed in claim 5, further characterized in that the addition of the DVB is in increments not exceeding 5 percent by weight of the total addition.

7. A process for making diethynylbenzene as claimed in any one of the preceding claims, further characterized in that between 50% to 80% of the divinylbenzene is a mixture of meta and para isomers of divinylbenzene.

8. A process for making diethynylbenzene as claimed in claim 7, further characterized in that between 78% to 80% of the divinylbenzene is a mixture of divinylbenzene isomers.

9. A process for making diethynylbenzene as claimed in any one of the preceding claims, further characterized in that a phase transfer agent in an amount of 0.5% to 3.0% by weight of the the reaction mixture from the bromination step, including the weight of the caustic agent, is added to the reaction mixture.

10. A process for making diethynylbenzene as claimed in claim 9, further characterized in that the phase transfer agent is a polyethylene glycol.

11. A process for making diethynylbenzene as claimed in any one of the preceding claims, further characterized in that the diethynylbenzene is recovered by steam distillation.

European Patent Office

# EUROPEAN SEARCH REPORT

Application Number

EP 91103225.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE - A - 2 146 845 (COSDEN OIL) * Examples 1,2,7; claims 1,7, 12,15 * -- | 1,4,7 | C 07 C 15/48<br>C 07 C 5/00 |
| A | SOVIET INVENTIONS ILLUSTRATED, CH section, week D 45, December 16, 1981 DERWENT PUBLICATIONS LTD., London, E 14 * SU-804 622 (NAIRIT POLYMER PROD) * -- | 1,9 | |
| A | SOVIET INVENTIONS ILLUSTRATED, CH section, week 8551, February 5, 1986 DERWENT PUBLICATIONS LTD., London, E 14 * SU-1159 917 (HETERO ORANIC CPDS) * -- | 1 | TECHNICAL FIELDS SEARCHED (Int. Cl.5) |
| A | EP - A1 - 0 194 798 (MITSUBISHI PETROCHEMICAL) * Page 23, line 18 - page 25, line 9 * ---- | 1 | C 07 C 15/00<br>C 07 C 5/00 |

The present search report has been drawn up for all claims

| Place of search | Date of completion of the search | Examiner |
|---|---|---|
| VIENNA | 02-07-1991 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)